**Europäisches Patentamt**

(19) **European Patent Office**

**Office européen des brevets**

(11) Veröffentlichungsnummer: **0 044 459**
**A2**

(12) **EUROPÄISCHE PATENTANMELDUNG**

(21) Anmeldenummer: **81105235.6**

(22) Anmeldetag: **06.07.81**

(51) Int. Cl.³: **A 61 F 13/04**

(30) Priorität: **18.07.80 DE 3027186**

(43) Veröffentlichungstag der Anmeldung:
**27.01.82 Patentblatt 82/4**

(84) Benannte Vertragsstaaten:
**AT BE CH DE FR GB IT LI NL SE**

(71) Anmelder: **BAYER AG**
**Zentralbereich Patennte, Marken und Lizenzen**
**D-5090 Leverkusen 1, Bayerwerk(DE)**

(72) Erfinder: **Burgdörfer, Hans-Heribert, Dr.**
**Goffineweg 49**
**D-5000 Koeln 80(DE)**

(72) Erfinder: **Lehnert, Günther, Dr.**
**c/o Cutter Laboratories, Inc. 2200 Powell Street**
**Emeryville California 94608(US)**

(54) **Orthopädischer Gehstollen für Stützverbände am Fuss.**

(57) Orthopädischer Gehstollen zur Verwendung bei Stützverbänden am Fuß, bestehend aus einem im wesentlichen quaderförmigen Stützkörper mit einer convexen, als·Trittfläche ausgebildeten Abrollfläche und darin befindlichen Einschnitten zur Fixierung am Stützverband, wobei an der fußseitigen Oberfläche des Stützkörpers eine innerhalb dieser Fläche versetzbare zweiseitig convex gekrümmte Pelotte angebracht ist.

Die Pelotte ist vorzugsweise am Stützkörper nach Art einer Steckverbindung mittels an der Pelottenunterseite angeordneten Zapfen befestigt, die mit längsseitig angeordneten Löchern in der Quaderoberfläche korrespondieren.

FIG. 2

EP 0 044 459 A2

BAYER AKTIENGESELLSCHAFT       5090 Leverkusen, Bayerwerk
Zentralbereich
Patente, Marken und Lizenzen  Ki/kl-c

17. Juli 1980

Orthopädischer Gehstollen für Stützverbände am Fuß

Die Erfindung betrifft einen orthopädischen Gehstollen zur Verwendung bei Stützverbänden am Fuß. Ein derartiger Gehstollen oder Laufklotz besteht aus einem im wesentlichen quaderförmigen Stützkörper mit einer convexen, als Trittfläche ausgebildeten Abrollfläche, die Einschnitte zur Fixierung des Gehstollens am Stützverband aufweist.

Unter "Stützverband" wird der konventionelle Gipsverband oder die in neuerer Zeit eingeführten Kunststoffverbände, beispielsweise auf Polyurethanbasis, verstanden. An diesem Stützverband wird in Höhe der Fußsohle üblicherweise ein Gehstollen angebracht, um dem Patienten das Gehen zu ermöglichen. Dabei soll der Patient trotz des Kunststoff- oder Gipsverbandes möglichst normal gehen können, d.h. über Ferse, Ballen und Zehen abrollen. Zu diesem Zweck sind bereits verschiedene Laufklötze bzw. Gehstollen beschrieben worden (siehe z.B. DE-PS 922 010, DE-OS 2 032 540 und US-PS 33 07 536).

BAD ORIGINAL

Die üblichen Gummistollen bringen aus orthopädischer Sicht diverse Probleme mit sich. Insbesondere bei den wenig auftragenden, leichten Kunststoffverbänden ist die sichere Positionierung und Fixierung des Stollens schwierig.

Der Erfindung liegt daher die Aufgabe zugrunde, einen Gehstollen zu entwickeln, der nicht nur in Verbindung mit den relativ gut modellierbaren Gipsverbänden sondern auch mit den modernen Kunststoffverbänden den orthopädischen Erfordernissen individuell angepaßt werden kann.

Diese Aufgabe wird erfindungsgemäß dadurch gelöst, daß an der fußseitigen Oberfläche des Stützkörpers eine innerhalb dieser Fläche versetzbare, zweiseitig convex gekrümmte Pelotte angebracht ist.

Zweckmäßig sind zur Befestigung der Pelotte an der fußseitigen Quaderoberfläche an der Pelottenunterseite Zapfen angeordnet, die mit längsseitigen Löchern in der Quaderoberfläche korrespondieren. Auf diese Weise kann die Pelotte mit den Zapfen an der erforderlichen Stelle in die Löcher eingesteckt werden. Die Löcher sind in einem dem Zapfenabstand entsprechenden Abstand auf einer Geraden parallel zur Längsrichtung angeordnet. Wenn die Pelotte nur in der Längsrichtung angepaßt zu werden braucht, genügt eine einzige Lochreihe.

- 3 -

Vorzugsweise hat die Pelotte die Form einer Kugel- oder Elipsoidkalotte und besteht zweckmäßig aus dem gleichen Material wie der Stützkörper. Besonders gute Eigenschaften werden erzielt, wenn Pelotte und Stützkörper aus einem elastischen, abriebfesten Polyurethanschaum hergestellt sind.

Der erfindungsgemäße Gehstollen ermöglicht es dem Fachmann (z.B. Orthopäden), das Fußgewölbe zu unterstützen und gleichzeitig den Gehstollen seitlich und in Längsrichtung exakt am Fuß zu positionieren. Der symmetrische Aufbau gestattet die Verwendung desselben Stollens für sowohl das rechte, wie das linke Bein. Darüber hinaus hat der neue Gehstollen den Vorteil, daß er sehr leicht, elastisch und abriebfest ist und mit Hilfe der Verschäumungstechnik wirtschaftlich herzustellen ist. Aufgrund des geringen Gewichtes kann der Gehstollen auch leicht und sicher mit den letzten Lagen des Stützverbandes angewickelt und am Fuß fixiert werden.

Im folgenden wird ein Ausführungsbeispiel der Erfindung anhand von Zeichnungen näher beschrieben. Es zeigen:

Figur 1    eine Draufsicht auf den Gehstollen von der Unterseite her (Trittfläche),

Figur 2    eine Seitenansicht, wobei die Trittfläche nach oben zeigt,

Le A 19 718

- 4 -

Figur 3    eine Draufsicht (Blickrichtung A in Figur 2)
           auf die dem Fuß zugewandte Stützkörperoberfläche und

Figur 4    eine Frontansicht (Blickrichtung B in Figur 2)
           wobei die Pelotte abgezogen ist.

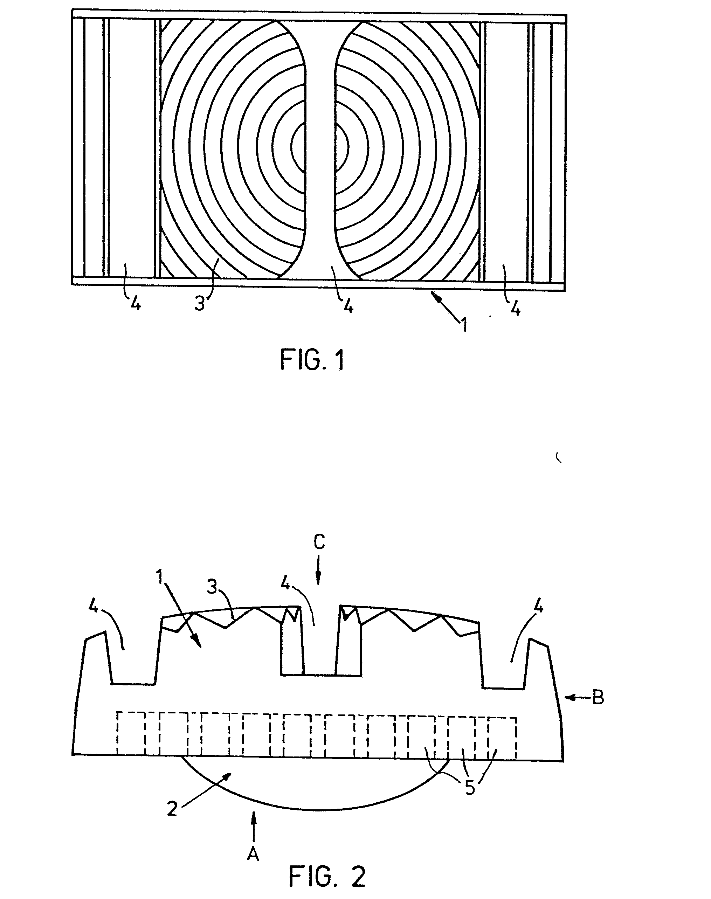

Der Gehstollen besteht aus einem im wesentlichen quaderförmigen Stützkörper 1 und einer Pelotte 2, die an der
Quaderoberfläche befestigt werden kann (siehe Figur 2
und Figur 4). An seiner Unterseite (Blickrichtung C in
Figur 2) ist der Stützkörper mit einem konzentrischen
Rillenprofil 3 versehen, um die Rutschsicherheit zu gewährleisten. Außerdem ist diese Seite in Längsrichtung
convex gewölbt, so daß die Trittfläche eine Abrollfläche bildet, wobei die Krümmung dem Abrollradius des
fixierten Gliedes entspricht. Quer zur Längsrichtung
sind in den Stützkörper 1 drei Einschnitte oder Nuten 4
eingearbeitet, die das Anwickeln des Gehstollens mit
den letzten Lagen des Stützverbandes ermöglichen und
damit eine sichere Fixierung des Gehstollens am Fuß gewährleisten.

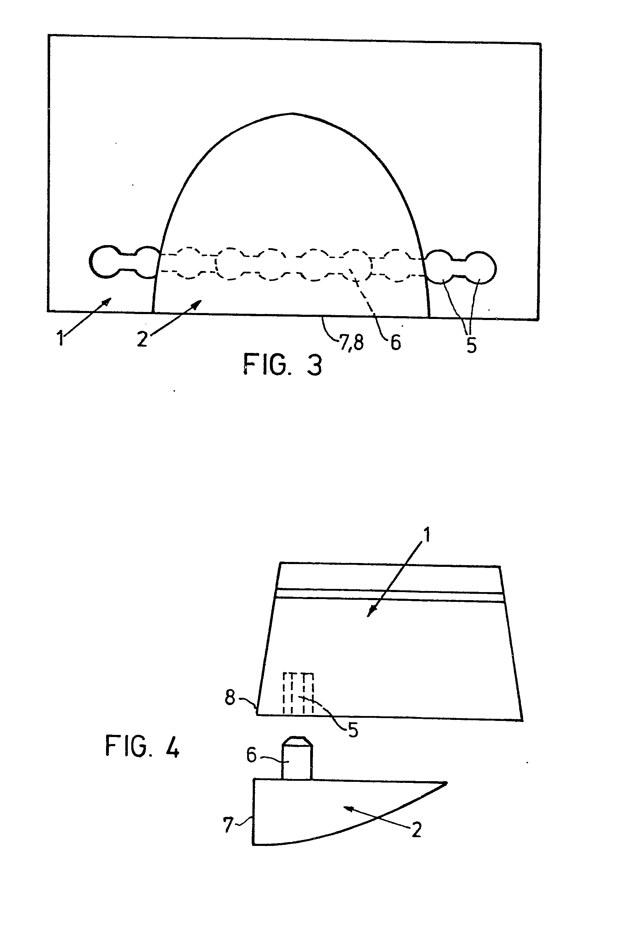

An der Planaren, der Trittfläche gegenüberliegenden Oberseite des Stützkörpers 1 ist eine zur Längsrichtung parallele Reihe von Löchern 5 angeordnet (Figur 2 und Figur 3). Die Befestigung der schon erwähnten Pelotte 2
an der Stützkörperoberfläche erfolgt nun in der Weise,
daß die Pelotte 2 mit einem oder mehreren Zapfen 6 ver-

Le A 19 718

sehen ist, die genau in die Löcher 5 passen (siehe Figur 4). Die Vielzahl der Löcher 5 erlaubt eine veränderliche Anpassung der Pelotte 2 in Längsrichtung; d.h. die Pelotte 2 kann nach vorne oder hinten versetzt oder wie in Figur 2 und Figur 3 genau in der Mitte eingesteckt werden. Welche Stelle gewählt wird, hängt jeweils von dem orthopädischen Befund ab. Im allgemeinen reicht es aus, wenn die Pelotte 2 nur in der Längsrichtung versetzt werden kann. Grundsätzlich besteht jedoch die Möglichkeit, mehrere Lochreihen 5 nebeneinander anzuordnen, so daß auch in der Querrichtung eine Anpassung der Pelotte 2 erfolgen kann. Durch diese Art der Anpassung wird seitlich und in Längsrichtung eine exakte Positionierung des Gehstollens und damit eine optimale Unterstützung des Fußgewölbes gewährleistet.

Die geometrische Form der Pelotte entspricht einer Kugel- oder Ellipsoidkalotte, die gemäß Figur 3 und Figur 4 in der Mitte durchgeschnitten ist. Die Schnittfläche 7 schließt dabei bündig mit der Seitenfläche 8 (Figur 3) ab.

Gefertigt wird der Gehstollen vorzugsweise, wie schon erwähnt, aus einem Polyurethanschaum, der abriebfest ist, eine gewisse Elastizität hat und im Unterschied zu den üblichen Stollenmaterialien sehr leich ist. Letztere Eigenschaft ist besonders in Verbindung mit den modernen Leichtkunststoff-Verbänden wichtig. Polyurethanschäume dieser Art sind an sich bekannt und werden z.B. in den Deutschen Offenlegungsschriften 2920 502, 29 31 469, 30 12 125 sowie 30 29 272 sowie der dort zitierten Literatur beschrieben.

Le A 19 718

Ausführungsbeispiel:

In eine Negativform, entsprechend dem oben beschriebenen Gehstollen-Modell, werden über ein Mischaggregat insgesamt 105 g folgender Komponenten eingetragen:

49 Gew.-% eines NCO-Vorpolymeren mit einem Isocyanatgehalt von 19 Gew.-% aus 50 Teilen 4,4'-Diphenylmethandiisocyanat, 10 Teilen Carbodiimidisiertem 4,4'-Diphenylmethandiisocyanat und 40 Teilen eines Polyesters aus Adipinsäure, Äthylenglykol und Butandiol (Molekulargewicht 2000)

51 Gew.-% einer Polyolformulierung gemäß nachfolgender Rezeptur:

100 Gewichtsteile des obigen linearen Polyesters, 15 Gewichtsteile eines Vernetzergemisches aus Äthylenglykol und Diäthylenglykol (Hydroxylzahl 1470; Wassergehalt 2,5 %) 0,4 Gewichtsteile Diazabicyclooctan

Nach einer Steigzeit von ca. 60 s und einer Formstandzeit von ca. 4 min. können der Gehstollen sowie die dazugehörige Palotte entnommen werden.

Die Rohdichte des Materials beträgt 540 kg/m$^3$; Zugfestigkeit ca. 6,5 MPa; Bruchdehnung 300 - 350 %; Abrieb bei 9,81 N Belastung und 40 m Schleifweg 100 - 200 mg. Im Praxisversuch wurden solche Stollen bis zu 6 Wochen lang getragen. Auch nach der längsten Tragedauer war das Rillenprofil noch deutlich zu erkennen.

Le A 19 718

Patentansprüche

1. Orthopädischer Gehstollen zur Verwendung bei Stützverbänden am Fuß, bestehend aus einem im wesentlichen quaderförmigen Stützkörper (1) mit einer convexen, als Trittfläche ausgebildeten Abrollfläche
und darin befindlichen Einschnitten (4) zur Fixierung am Stützverband, dadurch gekennzeichnet, daß
an der fußseitigen Oberfläche des Stützkörpers (1)
eine innerhalb dieser Fläche versetzbare zweiseitig, convex gekrümmte Pelotte (2) angebracht ist.

2. Gehstollen nach Anspruch 1, dadurch gekennzeichnet,
daß zur Befestigung der Pelotte (2) an der fußseitigen Quaderoberfläche nach Art einer Steckverbindung an der Pelottenunterseite Zapfen (6) angeordnet sind, die mit längsseitig angeordneten Löchern
(5) in der Quaderoberfläche korrespondieren.

3. Gehstollen nach Anspruch 2, dadurch gekennzeichnet,
daß mindestens eine Lochreihe (5) in Längsrichtung
vorgesehen ist.

4. Gehstollen nach Anspruch 1 bis 3, dadurch gekennzeichnet, daß die Pelotte (2) eine kugel- oder
Elipsoidkalotten-ähnliche Form aufweist.

5. Gehstollen nach Anspruch 1 bis 4, dadurch gekennzeichnet, daß Stützkörper (1) und Pelotte (2) aus
einem elastischen, abriebfesten Polyurethanschaum
gefertigt sind.

Le A 19 718

FIG. 1

FIG. 2

FIG. 3

FIG. 4